(19) Europäisches Patentamt
European Patent Office
Office européen des brevets

(11) Numéro de publication : **0 161 138 B1**

(12) # FASCICULE DE BREVET EUROPÉEN

(45) Date de publication du fascicule du brevet : 07.03.90

(51) Int. Cl.[5] : **G 01 N 33/53**, G 01 N 33/548// C12Q1/32

(21) Numéro de dépôt : **85400585.7**

(22) Date de dépôt : **26.03.85**

(54) **Procédé de dosage immunoenzymatique sans étape de séparation et réactifs et nécessaires pour sa mise en oeuvre.**

(30) Priorité : **27.03.84 FR 8404758**

(43) Date de publication de la demande : **13.11.85 Bulletin 85/46**

(45) Mention de la délivrance du brevet : **07.03.90 Bulletin 90/10**

(84) Etats contractants désignés : **AT BE CH DE FR GB IT LI LU NL SE**

(56) Documents cités :
**EP-A- 0 032 286**
**EP-A- 0 075 379**
**EP-A- 0 093 613**
**EP-A- 0 094 777**
**FR-A- 2 235 368**
**FR-A- 2 374 643**
**GB-A- 2 095 830**

(73) Titulaire : **INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur : **Nicolas, Jean-Claude**
**Le Phaéton Avenue du Père Soulas**
**F-34100 Montpellier (FR)**
Inventeur : **Terouanne, Béatrice**
**1, rue Henri**
**F-34000 Montpellier (FR)**
Inventeur : **Carrie, Marie-Luce**
**46, rue Frédéric Fabrèges**
**F-34000 Montpellier (FR)**
Inventeur : **Crastes de Paulet, André**
**12, avenue Lepic**
**F-34000 Montpellier (FR)**

(74) Mandataire : **Ayache, Monique et al**
**CABINET PLASSERAUD 84, rue d'Amsterdam**
**F-75009 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

## Description

L'invention a pour objet un procédé de dosage immunoenzymatique sans étape de séparation du marqueur libre de la fraction de marqueur liée, applicable au dosage de toute entité capable de former un complexe immunologique avec une autre entité qui lui est spécifique, ainsi que des réactifs et nécessaires pour essais, ou « kits », pour la mise en œuvre de ce procédé.

Les dosages immunoenzymatiques connaissent actuellement un essor important et différentes techniques ont été proposées. Elles utilisent soit des immunoadsorbants, comme le système connu sous la dénomination ELISA, soit la modification de l'activité biologique de l'enzyme utilisée comme marqueur, comme le système connu sous la dénomination EMIT. Cette dernière méthode présente l'avantage de ne pas nécessiter d'étape de séparation du marqueur libre de la fraction de marqueur liée, ce qui la rend facilement automatisable. Malheureusement, elle n'est applicable qu'au dosage des haptènes et sa sensibilité est relativement faible, ce qui limite considérablement son domaine d'application.

Pour tenter de remédier à ces inconvénients, on a proposé une méthode, en principe généralisable et ne nécessitant pas de séparation des formes libres et liées, qui consiste en la concentration de plusieurs enzymes au niveau d'un support, par l'intermédiaire d'une réaction immunologique. Dans un tel système, la substance produite par l'une des enzymes sert de substrat à une autre enzyme et on dose le produit final des différentes réactions mises en jeu (voir notamment Analytical Biochemistry 106, 223-229 (1980), demandes de brevets EP 0 032 286 et EP 0 075 379 et Immunoenzymatic Techniques, S. Avrameas et Coll., Elsevier Science Publishers B. V., (1983), page 247 et suivantes). Cependant, le signal obtenu selon cette technique évolue en fonction du temps. Par conséquent, toutes les mesures doivent être effectuées après un temps de réaction rigoureusement identique pour tous les dosages, dans une même série d'essais. Du fait des difficultés de mise en œuvre rencontrées ainsi que de la faible sensibilité et du manque de reproductibilité observés, cette méthode n'a pu être appliquée à des dosages de routine.

Dans ces documents, il n'est ni décrit, ni même suggéré, d'avoir recours, pour l'émission du signal, à un système de bioluminescence utilisant un nucléotide en tant que substance produite par la première enzyme.

On a par ailleurs [Analytical Biochemistry, 122, 385-393 (1982)] proposé une technique de dosage du dinitrophénol (DNP) ou du trinitrophénol (TNP) selon laquelle, au cours d'une première étape, on incube un conjugué de la glucose-6-phosphate deshydrogénase (G-6-PDH) et de l'haptène à doser modifié avec l'anticorps correspondant immobilisé et le composé libre à doser, on sépare la matière non liée, lave et ajoute du nicotinamide dinucléotide (NAD) et du glucose-6-phosphate (G-6-P). Il se forme du NAD réduit (NADH) selon la réaction :

$$\text{G-6-P} + \text{NAD}^+ \xrightarrow{\text{G-6-PDH}} \text{acide 6-phosphogluconique} + \text{NADH} + \text{H}^+.$$

Le NADH formé est dosé après incubation, au moyen de la lumière émise dans une réaction ultérieure, en ayant recours au système enzymatique bactérien de bioluminescence NADH : FMN (flavine mononucléotide) oxydo-réductase-luciférase, en présence de FMN et d'un aldéhyde à longue chaîne.

La lumière mesurée est produite selon les réactions suivantes :

$$\text{NADH} + \text{H}^+ + \text{FMN} \xrightarrow{\text{oxydo-réductase}} \text{NAD}^+ + \text{FMNH}_2 \quad \text{et}$$

$$\text{FMNH}_2 + \text{RCHO} + \text{O}_2 \xrightarrow{\text{luciférase}} \text{FMN} + \text{RCOOH} + \text{H}_2\text{O} + h\nu\,.$$

Dans ce procédé, on dose le NADH en solution. Pour augmenter le rendement de bioluminescence, il est nécessaire de laisser réagir au moins trente minutes pour que la quantité de NADH formée conduise à l'apparition d'une bioluminescence suffisante dans l'étape suivante.

Ce système donne des résultats décevants car le signal de bioluminescence obtenu est peu stable dans le temps, ce qui oblige à prendre des précautions particulières et constitue une source d'erreur importante. Au surplus, ce procédé est difficilement automatisable dans la mesure où il est nécessaire d'effectuer une séparation des composants non fixés avant d'effectuer le dosage par bioluminescence.

L'invention a pour but de remédier aux inconvénients des procédés de l'état de la technique en fournissant un procédé de dosage immunoenzymatique très sensible et présentant une bonne reproductibilité, applicable à tous les systèmes immunologiques et facilement automatisable, car ne nécessitant pas d'étape de séparation de la fraction contenant le marqueur en excès de la fraction contenant le complexe immun.

Ce but a été atteint selon l'invention qui, selon l'un de ses aspects, fournit un procédé qui satisfait aux exigences posées.

Selon l'invention, on a établi que, de façon surprenante, on pouvait obtenir un signal de

bioluminescence stable pendant une longue durée (plusieurs heures) avec un très bon rendement de bioluminescence, et partant une grande sensibilité, tout en observant une bonne reproductibilité, en concentrant, par l'intermédiaire d'une réaction immunologique, les différentes enzymes mises en jeu au niveau d'un support, et en travaillant dans des conditions particulières, et ceci même en présence de milieux biologiques.

Ces effets sont tout à fait inattendus, compte tenu d'une part de la complexité des milieux biologiques tels que le sérum qui contiennent notamment des enzymes, et d'autre part de l'instabilité connue des systèmes de bioluminescence.

Plus précisément, l'invention fournit un procédé pour le dosage immunoenzymatique, dans un milieu aqueux, notamment biologique, d'une entité X capable de former un complexe immunologique avec une entité Y, spécifique de l'entité X, selon lequel on effectue la concentration d'au moins deux enzymes au niveau d'un support au moyen d'une réaction immunologique à laquelle participent les entités X et Y, ce grâce à quoi un composé intermédiaire est formé au niveau du support par une première enzyme et selon lequel ce composé intermédiaire sert de substrat à une deuxième enzyme pour donner un autre composé qui, directement ou indirectement, produit au niveau du support un phénomène mesurable, dans des conditions telles qu'une relation puisse être établie entre le résultat de la mesure de ce phénomène et la concentration de l'entité X, caractérisé en ce qu'il comprend essentiellement les étapes consistant à :

a) amener une première enzyme, en tant que marqueur de l'entité X ou de l'entité Y, au moyen d'une réaction immunologique de type « compétition » ou « sandwich » entre les entités X et Y, au niveau du support sur lequel est fixée, de façon préalable ou concomitante, l'entité Y, pour y produire un composé nucléotide intermédiaire susceptible de subir, au contact des composants d'un système de bioluminescence comportant une ou plusieurs enzymes dont une au moins est également fixée sur le support, une transformation conduisant à l'émission de lumière, dans des conditions telles qu'une relation puisse être établie entre la lumière émise et la concentration de l'entité X dans le milieu aqueux,

b) laisser reposer jusqu'à obtention d'un signal stable,

c) mesurer la lumière émise et

d) à partir de la mesure effectuée, déterminer la concentration de l'entité X dans le milieu aqueux ou l'échantillon étudié, à l'aide d'un système d'étalonnage.

L'entité à doser X peut être tout composé pour lequel on sait préparer une entité spécifique Y avec laquelle il est capable de former un complexe immunologique. Dans le procédé selon l'invention, les entités X et Y peuvent en principe jouer des rôles symétriques, c'est-à-dire qu'en principe, une entité donnée X ou Y peut, selon le cas, être dosée au moyen de l'autre entité, ou servir à la doser.

Plus précisément, l'entité X est par exemple un antigène ou un haptène et l'entité Y est alors un anticorps spécifique de cet antigène ou de cet haptène, ou l'inverse.

Selon un mode avantageux de réalisation de l'invention, l'entité Y n'est elle-même fixée sur le support qu'au cours de la mise en œuvre du procédé selon l'invention, par l'intermédiaire d'une réaction immunologique avec une entité Z autre que l'entité X, fixée au préalable sur le support. Cette variante est particulièrement intéressante dans le cas du dosage d'un antigène ou d'un haptène. On utilise alors un support sur lequel est fixé au préalable un système permettant l'immobilisation ultérieure d'un anticorps, par exemple des anticorps anti-$\gamma$-globulines (on a alors un système dit « à double anticorps »), de la protéine A (protéine obtenue à partir de microorganismes tels que Staphylococcus aureus et utilisée pour la reconnaissance universelle des immunoglobulines) et analogues. L'anticorps spécifique de l'entité à doser est dans ce cas introduit dans le milieu aqueux au cours de la mise en œuvre du procédé et participe à une réaction immunologique avec d'une part l'entité Z fixée sur le support et d'autre part l'entité à doser X.

Le procédé selon l'invention peut être mis en œuvre en utilisant une réaction immunologique soit de type « compétition », soit de type « sandwich ». Ces deux expressions sont expliquées ci-après en relation, pour simplifier l'exposé, avec le dosage d'un antigène (composé X).

Réaction de type « compétition » : la première enzyme (marqueur) est fixée sur de l'antigène X plus ou moins modifié mais gardant des sites actifs reconnaissables par l'anticorps Y fixé sur le support (au préalable ou au cours de la réaction — voir plus haut). L'antigène X marqué par l'enzyme est mis en compétition avec l'antigène X à doser présent dans le milieu réactionnel, pour occuper les sites actifs de l'anticorps Y sur le support.

Réaction de type « sandwich » : dans ce cas, la première enzyme (marqueur) est fixée sur un anticorps spécifique de l'antigène à doser ; cet anticorps est le même que celui fixé sur le support, ou il est différent. L'antigène à doser forme un complexe immun avec d'une part l'anticorps spécifique fixé sur le support et d'autre part l'anticorps spécifique marqué par l'enzyme. Dans ce cas, la quantité d'enzyme ainsi fixée au voisinage du support, et partant la quantité de composé nucléotide intermédiaire formée et la quantité de lumière émise, sont en relation directe avec la concentration de l'antigène dans le milieu réactionnel. On peut donc parler ici de « dosage direct ».

Le composé nucléotide intermédiaire, appelé ci-après « composé intermédiaire », est formé directement ou indirectement par réaction de la « première enzyme » sur un substrat présent dans le milieu, de préférence en solution.

On entend ici par « système de bioluminescence » un système qui comprend essentiellement au moins une enzyme, luciférase, d'origine biologique et est capable de produire une émission de lumière par action sur le composé intermédiaire formé par la première enzyme. Ce système comprend également, si nécessaire, les constituants, composés chimiques ou enzymes, notamment, qui permettent de transformer le composé intermédiaire en substrat de la luciférase.

La luciférase et les autres enzymes éventuelles du système de bioluminescence sont fixées sur le support. Les composés chimiques éventuellement nécessaires sont apportés, de préférence en solution, au cours de la mise en œuvre du procédé.

Dans ce procédé, le choix de la luciférase du système de bioluminescence détermine le choix de la ou des autre(s) enzyme(s) et de leurs substrats. Cette notion sera explicitée ci-après à l'aide d'exemples.

Ainsi, la luciférase de luciole utilise, en tant que nucléotide, de l'ATP pour émettre de la lumière, selon les réactions simplifiées :

$$LH_2 + ATP \xrightarrow{\text{luciférase (E)}} E.\ LH_2AMP + PP$$

$$E.\ LH_2AMP + O_2 \rightarrow E.\ P + CO_2 + AMP + h\nu$$

il faut donc dans ce cas que l'enzyme fixée au niveau du support par la réaction immunologique, en présence des substrats appropriés, produise de l'ATP en tant que composé intermédiaire. Ainsi, par exemple, dans ce cas, la « première enzyme » peut être la pyruvate kinase et son substrat est l'ADP. Par ailleurs, on a vu que les luciférases bactériennes utilisent du NADH et du FMN en présence d'une oxydoréductase et d'un aldéhyde à longue chaîne. Ces luciférases peuvent également utiliser du NADPH. Lorsqu'on utilise une luciférase bactérienne, il faut donc disposer d'un système qui produise du FMN, ou, de préférence, du NADH ou du NADPH en tant que composé intermédiaire. Dans ce cas, la « première enzyme » peut, suivant une forme de mise en œuvre de l'invention, être la glucose-6-phosphate deshydrogénase qui produit du NADH (ou du NADPH) à partir de glucose-6-phosphate et de $NAD^+$ (ou respectivement $NADP^+$) en solution comme indiqué plus haut. Suivant une variante, la « première enzyme » peut être la malate deshydrogénase ayant pour substrats du malate et du $NAD^+$ (ou $NADP^+$). La réaction mise en jeu est alors :

$$\text{malate} + NAD^+\ [\text{ou } NADP^+] \xrightarrow[\text{deshydrogénase}]{\text{malate}} \text{oxalacétate} + NADH\ [\text{ou } NADPH] + H^+.$$

D'autres systèmes peuvent être imaginés par l'homme du métier sur la base de ses connaissances en la matière sans sortir du cadre de l'invention. En particulier, dans les cas indiqués plus haut, des systèmes plus complexes peuvent être imaginés pour produire le composé intermédiaire ATP, NADH ou NADPH. Dans tous les cas, les conditions du dosage (réactifs et proportions notamment) doivent être telles qu'une relation existe entre la quantité de composé intermédiaire formée et la quantité d'enzyme utilisée comme « marqueur » (première enzyme) fixée au niveau du support par l'intermédiaire de la réaction immunologique.

Le support peut être constitué de tout matériau qui n'absorbe pas, ou n'absorbe que très peu, la lumière et sur lequel il est possible de fixer, sans perte d'activité notable, la ou les enzyme(s) choisie(s) autre(s) que l'enzyme servant de marqueur, et l'entité Y ou une entité Z capable de fixer ultérieurement, au moyen d'une réaction immunologique, l'entité Y.

Pour obtenir un meilleur « confinement » des réactions qui se produisent au niveau du support, il peut être avantageux qu'il soit constitué d'une matière poreuse ou fibreuse.

Il peut ainsi par exemple être constitué de cellulose, d'acrylamide, de résine acrylique, d'agarose ou de dextrane.

Ce support peut se présenter sous forme d'une surface sensiblement plane (film, paroi d'un tube par exemple) ou sous forme d'une microbilles ayant jusqu'à environ 1 mm, de préférence environ 200 μ, de diamètre.

Dans le cas où on utilise des microbilles, les différents éléments (enzyme(s) et entité Y ou Z) peuvent être fixés sur des microbilles différentes, à condition toutefois que ces différents éléments soient dans la proximité immédiate les uns des autres grâce à un mélange intime.

Dans ce cas, bien que cela ne présente pas a priori un grand intérêt, on peut prévoir d'introduire les microbilles portant la ou les enzyme(s) du système de bioluminescence après avoir effectué la réaction immunologique et avant l'introduction des différents substrats.

Les matériaux à base d'agarose modifiée, se présentant sous forme de microbilles, commercialisés sous la dénomination de Sepharose par la Société Pharmacia, constituent des supports avantageux pour la mise en œuvre du procédé selon l'invention.

Quand tous les participants aux différentes réactions ont été ajoutés au milieu aqueux (leur ordre d'introduction dépend essentiellement des types de réactions enzymatiques et immunologiques mises en jeu), on laisse reposer jusqu'à obtention d'un signal stable (en général un repos de 10 minutes environ suffit), puis on effectue la mesure de la lumière émise.

Cette mesure peut être effectuée au moyen d'un luminomètre tel que, par exemple, celui commercialisé sous la dénomination LKB 1251 par la Société LKB.

Le système d'étalonnage peut être, entre autres, une courbe d'étalonnage représentant les variations de l'intensité de la lumière émise ou d'une valeur qui en dérive, en fonction de la concentration en entité X.

Une telle courbe peut être obtenue à partir de mesures avec des échantillons de concentration connue en entité X, effectuées dans des conditions similaires à celles du dosage.

La première enzyme est fixée sur l'entité X ou Y selon des méthodes usuelles bien connues de l'homme du métier. Plus de détails seront donnés à ce sujet dans les exemples qui suivent, en relation avec un mode particulier de fixation.

Le composé intermédiaire produit au niveau du support à l'aide de la première enzyme alimente directement les réactions de bioluminescence. Le même composé produit dans la solution aqueuse par la même enzyme non liée au support est en général suffisamment éloigné de la ou des enzyme(s) du système de bioluminescence pour ne pas interférer avec le dosage, ou n'interférer que d'une façon négligeable. Toutefois, selon un mode préféré de réalisation, on introduit dans le milieu un système, de préférence enzymatique soluble, capable de transformer le composé intermédiaire présent dans la solution en un composé non susceptible de donner lieu à l'émission de photons en présence du système de bioluminescence, sans modifier de façon importante le composé intermédiaire formé au niveau du support. A titre illustratif, on peut indiquer par exemple que lorsque le composé intermédiaire est du NADH, on peut le réoxyder dans la solution par un système enzymatique soluble tel que par exemple le système lactate déshydrogénase (LDH)-pyruvate. La réaction qui intervient est la suivante :

$$\text{NADH} + \text{pyruvate} \xrightarrow{\text{LDH}} \text{NAD} + \text{lactate.}$$

Selon un autre mode de réalisation, la FMN oxydo-réductase est utilisée en solution. Ainsi elle détruit le NADH en solution et alimente simultanément en $FMNH_2$ la luciférase immobilisée à partir du NADH formé au niveau du support, alors que le $FMNH^2$ produit en solution est spontanément oxydé.

Selon un mode préféré de réalisation de l'invention, le composé nucléotide intermédiaire est choisi parmi l'ATP, le NADH ou le NADPH.

Selon un mode tout particulièrement préféré de réalisation de l'invention,

la luciférase utilisée est d'origine bactérienne,

le composé intermédiaire est du NADH et

la première enzyme est la glucose-6-phosphate-déshydrogénase.

Selon un mode préféré de mise en œuvre du procédé selon l'invention, pour le dosage d'un antigène ou d'un haptène,

a) on fait incuber ensemble :

l'échantillon à doser,

une solution de l'entité à doser marquée par la première enzyme, et

une solution d'anticorps spécifique de l'entité à doser ;

b) on laisse réagir ;

c) on ajoute une suspension de Sepharose portant un système permettant l'immobilisation de l'anticorps en solution et la ou les enzyme(s) du système de bioluminescence et un « anticorps universel », de préférence un anticorps anti-γ-globulines ou une protéine A ;

d) on laisse de nouveau réagir ;

e) on ajoute, en solution dans un tampon, les substrats d'enzymes autres que le composé intermédiaire et de préférence les agents nécessaires à l'élimination du composé intermédiaire en solution ;

f) on laisse reposer jusqu'à obtention d'un signal stable de bioluminescence ;

g) on effectue la lecture de bioluminescence et

h) on détermine la concentration de l'entité à doser au moyen d'un système d'étalonnage.

On entend ici par « anticorps universel » un système permettant de fixer ultérieurement l'anticorps dans le but d'adapter ensuite ce système au dosage envisagé. Ce système améliore en outre la reproductibilité du dosage.

Il peut être avantageux d'utiliser, en tant qu'anticorps spécifique, un anticorps monoclonal.

Selon un mode tout particulièrement préféré de mise en œuvre du procédé selon l'invention, pour le dosage d'un antigène :

a) on fait incuber ensemble :

l'échantillon à doser,

un anticorps spécifique de l'entité à doser, marqué par la première enzyme,

un support portant, immobilisés, un anticorps spécifique de l'entité à doser, de préférence différent du précédent, et la ou les enzyme(s) du système de bioluminescence ;

b) on laisse incuber,

c) on ajoute, en solution dans un tampon, les substrats d'enzymes autres que le composé intermédiaire et, de préférence, les agents nécessaires à l'élimination du composé intermédiaire en solution ;

d) on laisse reposer jusqu'à obtention d'un signal stable de bioluminescence ;

e) on effectue la lecture de bioluminescence et

f) on détermine la concentration de l'entité à doser au moyen d'un système d'étalonnage.

Ce mode de réalisation de l'invention permet de réaliser un dosage très sensible car la relation entre la concentration de l'entité à doser et l'intensité lumineuse est pratiquement linéaire.

De préférence, on utilise dans ce dosage deux anticorps différents, spécifiques de deux parties différentes de la molécule d'antigène. Il peut être dans certains cas avantageux que l'un des anticorps soit monoclonal, voire les deux.

L'invention a également pour objet des réactifs pour la mise en œuvre du procédé selon l'invention.

Un tel réactif est caractérisé en ce qu'il se présente sous la forme d'un solide, de préférence à l'état de microbilles de Sepharose, comportant, fixées à sa surface, une entité capable de former un complexe immunologique avec une autre entité et la ou les enzyme(s) d'un système de bioluminescence.

Le solide peut être par exemple la paroi d'un tube dans lequel sera effectuée la réaction, ou une « languette » que l'on plonge dans la solution aqueuse où est effectué le dosage. De préférence cependant, il se présente sous forme de microbilles de Sepharose. Ces microbilles peuvent avantageusement être agglomérées sous forme de comprimé enrobé, délitable dans l'eau, ou de suspension dans l'eau.

La fixation de l'entité capable de former un complexe immunologique avec une autre entité et des enzymes sur le support est de préférence effectuée de façon covalente, sur la base des connaissances usuelles de l'homme du métier en cette matière. Une description plus précise d'une telle fixation est donnée dans les exemples qui suivent.

Selon un mode préféré de réalisation, l'entité capable de former un complexe immunologique avec une autre entité est un « anticorps universel », de préférence un anticorps anti-γ-globulines ou une protéine A.

Selon un mode tout particulièrement préféré de réalisation, l'invention fournit un réactif, caractérisé en ce qu'il est constitué de microbilles de Sepharose portant, fixés à leur surface sur les mêmes microbilles ou sur des microbilles différentes, un « anticorps universel », de préférence un anticorps anti-γ-globulines ou une protéine A, une luciférase d'origine bactérienne et une oxydoréductase.

Cette oxydoréductase est avantageusement la FMN oxydoréductase ou une diaphorase microbienne.

Selon un autre mode tout particulièrement préféré de réalisation, l'invention fournit un réactif caractérisé en ce qu'il est constitué de microbilles de Sepharose portant, fixés à leur surface, sur les mêmes microbilles ou sur des microbilles différentes, un anticorps spécifique de l'antigène à doser, une luciférase d'origine bactérienne et une oxydoréductase.

De plus, la présente demande vise un « nécessaire » ou « kit » pour la mise en œuvre du procédé selon l'invention.

Ce nécessaire comprend essentiellement :

le réactif selon l'invention décrit plus haut, à l'abri de la lumière, et

un flacon contenant l'entité à doser ou une entité spécifique de celle-ci, marquée par une enzyme.

Selon un mode tout particulièrement préféré de réalisation, le nécessaire selon l'invention comprend :

un flacon contenant, à l'abri de la lumière, du Sepharose, en suspension dans l'eau, ou sous forme de comprimé délitable dans l'eau, muni à sa surface d'un « anticorps universel », d'une luciférase d'origine bactérienne et d'une oxydoréductase,

un flacon contenant un anticorps spécifique de l'antigène à doser,

un flacon contenant l'antigène à doser marqué par une oxydoréductase, et

du tampon, du NAD ou du NADP, du FMN, un aldéhyde à longue chaîne, un système capable de détruire le NADH ou le NADPH en solution ainsi que le substrat de l'oxydoréductase servant de marqueur.

Selon encore un autre mode tout particulièrement préféré de réalisation, le nécessaire selon l'invention comprend :

un flacon contenant, à l'abri de la lumière, du Sépharose en suspension dans l'eau ou sous forme de comprimé délitable dans l'eau, muni à sa surface d'une entité spécifique de l'entité à doser, d'une luciférase d'origine bactérienne et d'une oxydoréductase,

un flacon contenant l'entité à doser ou une entité spécifique de l'entité à doser, marquée par une oxydoréductase, et

du tampon, du NAD ou du NADP, du FMN, un aldéhyde à longue chaîne, un système capable de détruire le NADH ou le NADPH en solution, ainsi que le substrat de l'oxydoréductase servant de marqueur.

D'autres caractéristiques de l'invention apparaîtront à la lecture des exemples qui suivent donnés à titre purement illustratif.

Exemple 1

Préparation du réactif selon l'invention

Les enzymes du système de bioluminescence utilisées, luciférase et NADH [ou NADPH] réductase-FMN spécifique, appelée ci-après FMN oxydoréductase, d'origine bactérienne, sont préparées par culture de Beneckea Harveyi selon le procédé décrit par A. H. Farghaly dans J. Cell. Comp. Physiol. 36 (1969), 165-184 et purifiées selon la méthode de A. Gunsalus-Miguel et coll. décrite dans J. Biol. Chem. 247 (1972), 398-404, telle que modifiée par T. O. Baldwin et coll. dans J. Biol. Chem. 250 (1975) 2763-2768.

On couple 5 g d'agarose modifiée se présentant sous forme de microbilles, commercialisée par la Société Pharmacia sous la dénomination Sepharose 4B, activée par le bromure de cyanogène avec 10 mg de luciférase, 5 U.I. de FMN oxydoréductase et soit 5 mg de protéine A, soit 5 mg d'anticorps anti-γ-globulines, soit encore la quantité d'anticorps spécifique de l'antigène ou du haptène à doser, ou de l'antigène correspondant à l'anticorps à doser, suffisante pour 1000 dosages. Par exemple, dans le cas du dosage de la progestérone, on fixe 1 ml d'anticorps anti-progestérone correspondant à la quantité commercialisée pour 1000 dosages.

Après réaction, la phase solide est lavée avec un tampon phosphate 0,1 M, pH 6,8, contenant $10^{-3}$ M de dithiothréitol (DTT) et 0,1 % de $NaN_3$.

Ce réactif peut être conservé à l'abri de la lumière, en suspension dans l'eau ou sous forme de comprimé délitable dans l'eau.

Exemple 2

Préparation d'un haptène marqué par une enzyme

Couplage de la progestérone à la G-6-PDH.

a) Principe

De nombreux conjugués protéine-stéroïde ont été synthétisés par réaction d'un stéroïde alcoylant avec un résidu de cystéine. Cependant, la faible quantité de groupements SH dans la plupart des protéines présente un sérieux handicap pour le couplage. I. M. Klotz et coll. (Arch. Biochem. Biophys. 96 (1962), 605-612) ont décrit une méthode pour augmenter le nombre de groupements sulfhydryle par l'intermédiaire de résidus lysine, c'est cette méthode qui est utilisée.

De plus, une faible quantité d'haptène doit être liée à l'enzyme et l'activité enzymatique conservée. Généralement la modification de quelques groupements aminés des enzymes n'affecte pas leur activité spécifique. Enfin, pour augmenter la sensibilité du dosage, le bras reliant l'haptène à l'enzyme doit être différent de celui utilisé pour la préparation de l'immunogène (haptène fixé sur de la sérum albumine de bovins (BSA) par exemple) utilisé pour produire les anticorps spécifiques participant au dosage.

b) Introduction de résidus thiol sur l'enzyme

On incube pendant 1/2 h 1 mg de G-6-PDH dans 1 ml de tampon phosphate 0,05 M, pH 7,2 dans $5.10^{-3}$ M d'anhydride S-acétyl mercaptosuccinique (SAMSA) en présence de NADP $10^{-5}$ M pour protéger l'activité enzymatique. Le pH du milieu est suivi au cours de la réaction. La solution enzymatique est incubée pendant deux heures à 4 °C en présence de 300 µl de DTT $5.10^{-3}$ M et d'hydroxylamine $10^{-1}$ M, préparés avec du tampon phosphate 0,05 M, pH 7,2. L'enzyme thiolée est déposée sur une colonne de support pour chromatographie commercialisé sous la dénomination de Sephadex G 25, équilibrée avec du tris, HCl pH 8 0,2 M à 20 % de glycérol pour éliminer l'excès de réactif et amener l'enzyme modifiée à pH = 8.

Le nombre de groupements thiol libre est déterminé par la technique de G. L. Ellman et coll. (Arch. Biochem. Biophys. 82 (1959), 70-77).

Le 5,5'-dithiobis 2-nitrobenzoate (DTNB) réagit avec les groupements thiol de la G-6-PDH dans les conditions décrites ci-dessous.

Le coefficient d'absorption molaire du chromophore est égal à 13600 $M/cm^{-1}$. Les mesures d'absorption sont réalisées à 412 nm.

Le dosage est réalisé sur 50 µl de solution enzymatique éluée du Sephadex G 25 dans 1 ml de tampon tris, HCL 0,2 M pH 8.100 µl de DTNB ($10^{-2}$ M) sont ajoutés au mélange. L'absorbance de cette solution est déterminée à 412 nm contre un tampon tris, HCl 0,2 M pH 8, en présence du réactif d'Ellman.

c) Couplage de la G-6-PDH avec du 11 α-bromoacétate de progestérone

A sa sortie de la colonne de Sephadex G 25, la G-6-PDH thiolée est immédiatement couplée au 11 α-bromoacétate de progestérone ($10^{-4}$ M) préparé par couplage de la 11 α-hydroxyprogestérone avec l'acide bromoacétique activé par un carbodiimide.

Cette étape doit être effectuée sans attendre pour éviter l'oxydation des groupements SH. La réaction dure une nuit. Tout le stéroïde non lié est éliminé par passage de la solution enzymatique sur une colonne de Sephadex G 25.

## Exemple 3

### Préparation d'un anticorps marqué par une enzyme couplage d'un anticorps avec la G-6-PDH

a) Principe

Le couplage entre ces deux protéines est réalisé selon la méthode de M. J. O'Sullivan et coll. (Analytical Biochemistry, 100 (1979), 100-108) en utilisant un réactif bifonctionnel, l'ester N-hydroxysuccinimide de l'acide méta maléimido benzoïque (MBS).

b) Traitement des anticorps par le MBS

Le MBS est dissous dans le diméthylformamide (2 mg/ml), 200 µl de cette solution sont ajoutés à 1,5 mg d'anticorps en solution dans 1,5 ml de tampon phosphate, pH 7,2 et incubés 1 heure à 28 °C puis filtrés sur colonne de Sephadex G-25.

c) Couplage de l'anticorps avec la glucose-6-phosphate deshydrogénase

La deshydrogénase enrichie en groupements SH selon la méthode indiquée à l'exemple 2 (2 mg) est incubée pendant 2 heures à pH 7 avec les anticorps traités par le MBS. Après incubation, les différents conjugués sont séparés par chromatographie sur Ultrogel ACA34 (produit commercial de la Société IBF).

## Exemple 4

### Préparation d'un antigène marqué par une enzyme

Le couplage entre ces deux substances protéiques est réalisé selon la méthode décrite à l'exemple 3.

## Exemple 5

### Dosage d'un antigène ou d'un haptène par compétition

Le milieu d'incubation est composé de :

100 µl d'échantillon à doser,

100 µl de solution de l'antigène ou du haptène à doser marqué par la G-6-PDH (0,06 mU.I.) et

100 µl de solution d'anticorps spécifiques de l'antigène ou respectivement du haptène à doser, permettant de lier environ 50 % de l'entité marquée.

Après 15 minutes de réaction, 100 à 500 µl d'une suspension de réactif préparé à l'exemple 1, muni d'un « anticorps universel », sont ajoutés et la réaction est poursuivie pendant encore 15 minutes. La lecture de bioluminescence est réalisée sur un luminomètre du type de celui commercialisé sous la dénomination LKB 1251, 10 minutes après addition de 1 ml de tampon phosphate 0,1 M, à pH 7, contenant $10^{-5}$ M de FMN, $5.10^{-5}$ M de décanal, $2.10^{-3}$ M de glucose-6-phosphate, $10^{-3}$ M de NAD, $10^{-3}$ M de pyruvate et 10 à 1000 mU.I. de lactate deshydrogénase.

Les réactions mises en jeu sont :

dans la solution

Ag— DH + Ag + Ac ———> Ac -Ag— DH +Ac -Ag

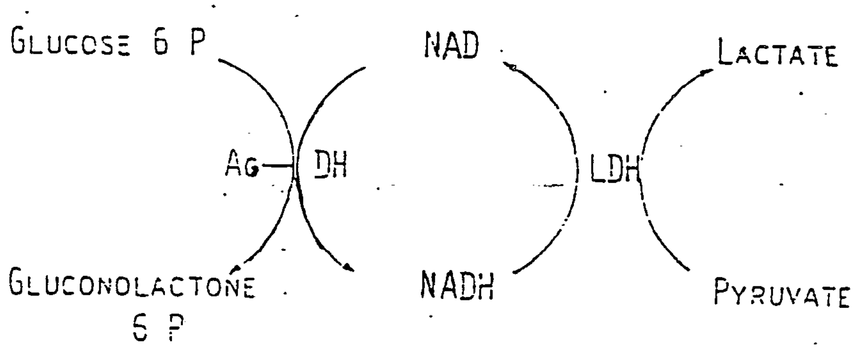

au niveau du support :

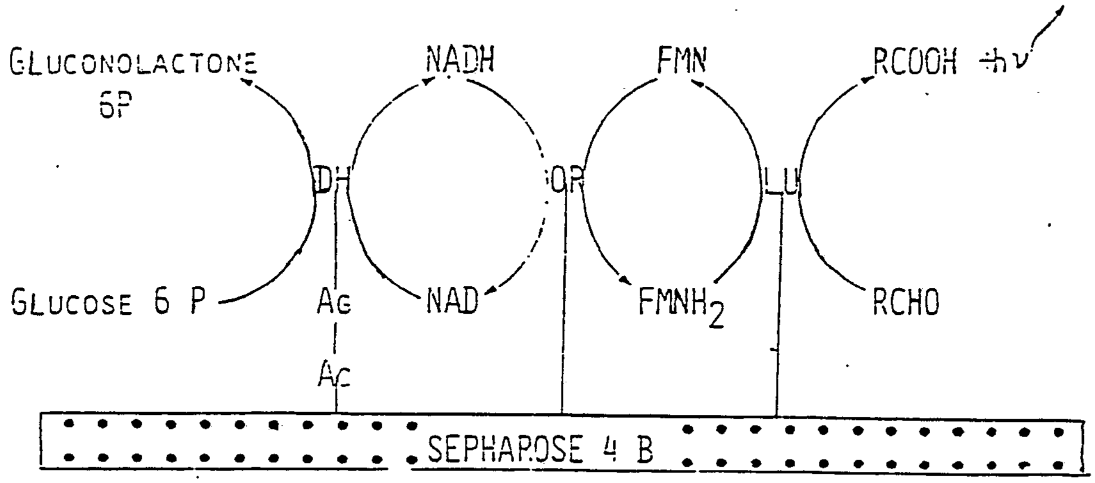


AG – DH = Antigène marqué par la glucose 6 P deshydrogénase
OR = NAD-FMN oxydoréductase
LU = Luciférase
LDH = Lactate deshydrogénase
AC = Anticorps spécifique
RCHO = Décanal

Application au dosage de la progestérone. Résultats

Dans ce cas, l'échantillon est, par exemple, un extrait de plasma ou de la salive.

L'étude est effectuée avec le réactif selon l'exemple 1 portant des anti-γ-globulines de lapin.

Le signal obtenu augmente rapidement en fonction du temps après addition du réactif, puis se stabilise. La concentration du marqueur G-6-PDH au niveau du gel d'agarose par l'addition d'un anticorps spécifique, conduit à une augmentation importante du signal lumineux : le signal est augmenté de 20 à 100 fois, mais, dans ce cas, seulement 20 % de l'activité enzymatique sont liés par l'anticorps ; le facteur d'amplification de luminescence obtenu est donc de : 20/0,2 = 100 à 100/0,2 = 500.

Lorsqu'on ajoute au tampon de la lactico-deshydrogénase qui, en présence de pyruvate, détruit le NADH formé par la G-6-PDH en solution, on obtient un signal stable pendant plusieurs heures du fait que les deux réactions d'oxydation et de réduction du NAD s'équilibrent rapidement en solution, évitant une augmentation de la quantité de NADH. Avantageusement, la lactico-deshydrogénase est présente dans le milieu en large excès.

Le tableau I montre les résultats obtenus pour différentes quantités de réactif selon l'exemple 1 : à partir de 6 mg, le signal maximum est atteint.

Tableau I

| Quantité de Sépharose portant Ac.L.R. (mg de gel essoré) | Luminescence maximale (pas de compétition) (mV) | Luminescence minimale (NS) (pas d'anticorps spécifiques) (mV) |
|---|---|---|
| 12,5 | 5700 | 470 |
| 6,25 | 5624 | 250 |
| 2,5 | 4443 | 160 |
| 1,25 | 3335 | 80 |

Ac : anticorps
L : luciférase
R : réductase
NS : non significatif.

Dans le cadre de cet exemple, on obtient une bonne reproductibilité avec une quantité de gel essoré comprise entre 6 et 12,5 mg environ.

On peut tracer une courbe d'étalonnage représentant les variations du rapport B/Bo (B : bioluminescence obtenue en présence de progestérone ; Bo : bioluminescence maximale en l'absence de progestérone) en fonction de la concentration en progestérone et déterminer au moyen de cette courbe une concentration inconnue en progestérone à partir de la mesure de B.

Les variations sont inférieures à 10 % (reproductibilité de la méthode supérieure à 10 %).

Les extraits de sérum à l'éther ne présentent pas d'interférences avec la réaction de bioluminescence.

En effet, en examinant le tableau II suivant on constate que les résultats obtenus pour le dosage de la progestérone dans des extraits éthérés de sérum sont indépendants du volume de l'échantillon.

Tableau II

| Volume de l'échantillon (µl) | Dosage sur extrait éthéré (ng/ml) |
|---|---|
| 10 | ♀ ... 4,20<br>♂ ... 0,36 |
| 20 | ♀ ... 4,1<br>♂ ... 0,4 |
| 50 | ♀ ... 4,3<br>♂ ... 0,38 |

De plus, en raison de sa grande sensibilité, le dosage peut être réalisé directement, sans extraction, sur 10 à 20 µl de sérum préalablement inactivé par la chaleur, en présence de 20 % d'alcool.

Le tableau III montre la reproductibilité des résultats dans cette gamme de volumes.

| Volume de l'échantillon (µl) | Dosage sur sérum complet, inactivé par la chaleur (ng/ml) |
|---|---|
| 10 | ♀ ... 4,1<br>♂ ... 0,3 |
| 20 | ♀ ... 4<br>♂ ... 0,36 |

Exemple détaillé

Description détaillée du dosage de la progestérone

A) Description du dosage

Ce dosage repose sur la compétition entre la progestérone à doser et la progestérone marquée par la glucose-6-phosphate deshydrogénase de Leuconostoc mésentéroides (G6PDH) vis-à-vis d'un nombre limité de sites anticorps. L'anticorps antiprogestérone utilisé est soit un anticorps polyclonal de lapin, soit un anticorps monoclonal de souris. Lors du dosage, l'anticorps spécifique est immunoadsorbé sur un support sur lequel sont insolubilisés un second anticorps anti-immunoglobuline de lapin ou de souris, la FMN oxydoréductase et la luciférase de Beneckea harveyi. On appellera ce support : l'adsorbant IEL (Immuno Enzymo Luminescent).

Le dosage comprend les étapes suivantes :

extraction de la progestérone dans les sérums à doser par de l'éther éthylique ;

incubation dans un tampon PBS BSA (tampon phosphate/sérumalbumine de bovins) pendant 30 minutes à 28 °C de 100 µl de standard ou d'échantillon à doser, 100 µl de progestérone marquée par la G6PDH ($30.10^{-6}$ UI par dosage) et 100 µl d'anticorps spécifique permettant de lier 40 % à 50 % de la progestérone marquée ;

incubation d'une heure dans un tampon PBS BSA sous agitation continue de ces solutions avec

300 µl de suspension d'adsorbant IEL (soit environ 2 mg de Sepharose par essai), préparé par couplage de 1 gramme de Sepharose activée au bromure de cyanogène à 2 mg de luciférase, 0,5 UI d'oxydoréductase et 2 mg de double anticorps anti lapin ou anti souris ;

Lecture de la bioluminescence sur un luminomètre LKB 1251 couplé à un ordinateur commercialisé sous la dénomination Apple II, 10 minutes après injection automatique dans chaque tube de 1 ml de réactif de bioluminescence composé de tampon PBS BSA contenant $10^{-5}$ M de FMN, $6.10^{-5}$ M de décanal, $3.10^{-3}$ M de glucose-6-phosphate, $10^{-3}$ M de NAD, $7.10^{-4}$ M de pyruvate et 10 mUI/ml de lactate deshydrogénase. Les lectures peuvent être faites plusieurs fois, compte tenu de la stabilité du signal.

B) Résultats

a) Stabilité du réactif

La luminescence de 24 dosages a été mesurée toutes les 10 minutes pendant 2 heures. Sur les 12 mesures effectuées pour chaque essai, la variation moyenne est égale à 0,6 % et les variations extrêmes sont égales au minimum à 0,2 % et au maximum à 1,5 %. Le réactif peut donc être préparé à l'avance et la lecture peut s'effectuer de 10 minutes à 2 heures après l'injection à condition de conserver les tubes et réactifs à l'obscurité.

b) Influence des liquides biologiques sur la mesure de la bioluminescence

Dans un tube contenant l'anticorps antiprogestérone, le marqueur et l'adsorbant IEL sont ajoutés, juste avant l'injection du réactif de bioluminescence, soit :

100 µl d'extrait de sérum desteroïdé
100 µl de salive congelée, centrifugée et chauffée à 60 °C
25 µl de sérum total desteroïdé.

Les résultats sont donnés dans le tableau ci-après :

| | tampon PBS BSA | extrait de sérum | salive | sérum total |
|---|---|---|---|---|
| luminescence | 100% | 99% | 93% | 90% |

Au vu de ces résultats, les dosages de la progestérone ont été faits sur des extraits de sérums.

c) Dosage de la progestérone

Deux courbes étalons du dosage de la progestérone ont été établies soit par RIA (essai radioimmunologique) avec la progestérone tritiée comme traceur, soit par bioluminescence. Une sensibilité tout à fait comparable a été obtenue : pour le dosage RIA, le coefficient de corrélation est égal à 0,996, l'équation de la droite est $Y = 1{,}82 - 0{,}98\,X$, 50 % de déplacement de la liaison du marqueur sont obtenus pour une valeur de 66 pg de progestérone. Avec le dosage immunoenzymatique bioluminescent (BEIA) selon l'invention, le coefficient de corrélation est égal à 0,994, l'équation de la droite est $Y = 1{,}97 - 0{,}98\,X$ et 50 % de déplacement sont obtenus pour 95 pg de progestérone.

Des sérums de femme en phase folliculaire ou en phase lutéale ont fait l'objet d'une double évaluation de leur teneur en progestérone, l'une par RIA et l'autre par BEIA. La corrélation entre ces deux dosages est très significative : $R = 0{,}971$, $y = 0{,}939\,X + 0{,}028$.

La précision du dosage BEIA a été testée en faisant des essais de répétabilité (intra essai) et de reproductibilité (inter essai).

Répétabilité du dosage BEIA (intra essai)

| Echantillon | concentration ng/ml | Ecart type | Coefficient de variation |
|---|---|---|---|
| I | 1,1 | 0,14 | 12% n=10 |
| II | 4,8 | 0,31 | 6,2% n=10 |
| III | 14,3 | 0,78 | 5,4% n=10 |

Reproductibilité du dosage BEIA (inter essai)

| | | | |
|---|---|---|---|
| I | 2,48 | 0,22 | 9% n=10 |
| II | 4,25 | 0,3 | 7% n=10 |
| III | 15 | 1,15 | 7,6% n=10 |

Exemple 6

Dosage d'une protéine par la méthode « sandwich » ou « dosage direct »

Le milieu d'incubation est composé de :
100 µl d'échantillon à doser,
100 µl d'anticorps spécifique de la protéine à doser, marqué par la G-6-PDH,
100 à 500 µl de réactif selon l'exemple 1 portant un anticorps spécifique de la protéine à doser.

De préférence, l'anticorps marqué par la G-6-PDH et l'anticorps fixé sur le support reconnaissent des parties différentes de la protéine. Il peut être avantageux que l'un au moins d'entre eux, sinon les deux, soit monoclonal.

Après 1 à 2 heures d'incubation, on ajoute 1 ml de tampon phosphate 0,1 M, pH 7, contenant $10^{-5}$ M de FMN, $5.10^{-5}$ M de décanal, $2.10^{-3}$ M de glucose-6-phosphate, $10^{-3}$ M de NAD, $10^{-3}$ M de pyruvate et 10 à 1000 ml.U. de lactate deshydrogénase. On laisse reposer 10 minutes et on lit la luminescence.

Applications de l'exemple 6

I. Utilisation d'anticorps non monoclonaux

a) Dosage de la gonadotrophine chorionique

La bioluminescence lue (en mV) varie de façon linéaire en fonction de la quantité de l'hormone dans l'essai (exprimée en mU.I.) jusqu'à 10 mU.I./ml. La limite de sensibilité est de 0,5 mU.I./ml.

b) Dosage des γ-globulines de souris :

On procède comme indiqué plus haut en utilisant des anticorps anti-γ-globulines et en laissant incuber deux heures.

On établit une gamme pour des concentrations en γ-globulines allant de 5 pM à 100 pM. La luminescence lue varie de 450 mV à 3000 mV, d'une façon linéaire en fonction de la concentration en γ-globulines.

II. Utilisation d'anticorps monoclonaux

Les dosages homogènes sans séparation étaient réservés jusqu'à présent aux dosages des petites molécules : médicaments, haptènes. Et les dosages de type « sandwich » ne pouvaient être envisagés sans plusieurs étapes de séparation et de lavage. Grâce au procédé selon l'invention qui présente une très bonne sensibilité au niveau de la détection du marqueur, et en utilisant des anticorps de très haute affinité, il a été possible de réaliser, avec une très bonne sensibilité, un dosage immunométrique sans étape de séparation de l'hormone chorionique gonatropine (HCG) et de l'α-foeto protéine (AFP).

A) Dosage de l'HCG

Le dosage de l'HCG a été réalisé en utilisant un anticorps monoclonal de souris anti-HCG marqué par la G6PDH. L'adsorbant a été synthétisé en couplant des anticorps polyclonaux de lapin anti-HCG, la luciférase et l'oxydo-réductase sur Sepharose activée au bromure de cyanogène.

Protocole du dosage

100 µl d'anticorps marqué par la G6PDH (0,16 mUI par essai) sont incubés avec 100 µl de standard d'HCG : 0, 25, 50, 100, 200, 400 pg dans l'essai. L'HCG (Boehringer) a une activité spécifique de 10000 UI par mg. Les standards sont préparés dans un tampon PBS BSA contenant 25 % de sérum total. Cette incubation dure 1 heure à 28 °C. L'incubation avec 300 µl d'adsorbant IEL dure 2 heures à température ambiante sous agitation continue. Les tubes sont ensuite introduits dans le luminomètre et la bioluminescence est mesurée comme il a été décrit précédemment pour le dosage de la progestérone. Les déterminations sont effectuées en reportant les valeurs lues sur une courbe étalon établie à partir des mesures effectuées pour les concentrations standard ; une très bonne sensibilité est obtenue même pour des valeurs inférieures à 1 ng/ml.

b) Dosage de l'AFP

Le même principe a été appliqué pour doser l'AFP. Des anticorps monoclonaux anti-AFP (liquide d'ascite) et des anticorps polyclonaux anti-AFP purifiés ont été utilisés. L'anticorps monoclonal purifié a été couplé à la G6PDH, les anticorps polyclonaux coimmobilisés sur Sepharose avec la luciférase et l'oxydo-réductase.

Protocole du dosage et résultats

100 µl d'anticorps monoclonal anti-AFP marqué par la G6PDH (0,32 mUI par essai) sont incubés 1 heure à 28 °C avec 100 µl d'échantillon. Les dilutions de standards sont faites dans un milieu PBS BSA contenant 25 % de sérum entier. La deuxième incubation avec l'adsorbant dure deux heures à température ambiante sous agitation continue. La courbe étalon du dosage de l'AFP montre que moins de 5 Ul/ml peuvent être détectées par ce dosage BEIA.

En conclusion :

Contrairement aux méthodes décrites jusqu'à présent, le dosage BEIA selon l'invention permet d'obtenir un signal stable pendant plusieurs heures, tout en évitant toute étape de séparation telle que centrifugation et lavage, ce qui permet de faciliter le dosage et d'améliorer la reproductibilité. Ce procédé est facilement automatisable car il nécessite seulement l'addition des réactifs, et peut s'appliquer aussi bien aux dosages de petites molécules que de protéines.

Le dosage de la progestérone réalisé selon ce procédé est aussi performant qu'un dosage de type RIA.

Quant aux dosages de l'AFP et de l'HCG, leur sensibilité est comparable à celle donnée par d'autres méthodes : dosage de l'AFP par immunochimiluminescence utilisant les esters d'acridinium (I. Weeks et coll., Clin. Chem. (1983), 29, 1480-1483) et dosage de l'HCG par immunofluorescence avec les chélates des terres rares (Europium) (K. Pettersson et coll., Clin. Chem. (1983), 29, 60-64). Ces résultats ont pu être obtenus grâce à l'utilisation d'anticorps monoclonaux de très haute affinité ; cette très bonne réactivité immunologique évite l'utilisation d'un excès d'anticorps marqué.

Ainsi, alors que les méthodes de dosage immuno-enzymatiques habituelles sont moins sensibles que les méthodes radioimmunologiques, le procédé de dosage immunoenzymatique selon l'invention présente une sensibilité tout à fait comparable qui peut être encore améliorée grâce à l'utilisation d'anticorps de haute spécificité.

De plus, ce dosage peut être mis en œuvre en présence de milieux biologiques. Ainsi, la bioluminescence est peu affectée par la présence de 25 µl de sérum total dans 1 ml de milieu de dosage.

## Revendications

1. Procédé pour le dosage immunoenzymatique, dans un milieu aqueux, notamment biologique, d'une entité X capable de former un complexe immunologique avec une entité Y, spécifique de l'entité X, selon lequel on effectue la concentration d'au moins deux enzymes au niveau d'un support au moyen d'une réaction immunologique à laquelle participent les entités X et Y, ce grâce à quoi un composé intermédiaire est formé au niveau du support par une première enzyme et selon lequel ce composé intermédiaire sert de substrat à une deuxième enzyme pour donner un autre composé qui, directement ou indirectement, produit au niveau du support un phénomène mesurable, dans des conditions telles qu'une relation puisse être établie entre le résultat de la mesure de ce phénomène et la concentration de l'entité X, caractérisé en ce que le support est poreux et en ce que ledit procédé comprend essentiellement les étapes consistant à :

a) amener une première enzyme, en tant que marqueur de l'entité X ou de l'entité Y, au moyen d'une réaction immunologique de type « compétition » ou « sandwich » entre les entités X et Y, au niveau du support poreux sur lequel est fixée, de façon préalable ou concomittante, l'entité Y, pour y produire à partir d'un composé nucléotide introduit dans le milieu en tant que réactif, un autre composé nucléotide, intermédiaire, susceptible de subir, au contact des composants d'un système de bioluminescence comportant une ou plusieurs enzymes dont une au moins est également fixée sur le support, une transformation conduisant à l'émission de lumière, dans des conditions telles qu'une relation puisse être établie entre la lumière émise et la concentration de l'entité X dans le milieu aqueux, ce milieu étant en outre additionné d'un système, de préférence enzymatique soluble, capable de transformer le composé nucléotide intermédiaire présent dans la solution en un composé non susceptible de donner lieu à l'émission de photons en présence du système de bioluminescence, sans modifier de façon importante le composé nucléotide intermédiaire formé au niveau du support,

b) laisser reposer jusqu'à l'obtention d'un signal stable,

c) mesurer la lumière émise et

d) à partir de la mesure effectuée, déterminer la concentration de l'entité X dans le milieu aqueux ou l'échantillon étudié, à l'aide d'un système d'étalonnage.

2. Procédé selon la revendication 1, caractérisé en ce que le composé nucléotide intermédiaire est choisi parmi l'ATP, le NADH ou le NADPH.

3. Procédé selon la revendication 2, caractérisé en ce que :

la luciférase utilisée est d'origine bactérienne,

le composé nucléotide intermédiaire est du NADH,

le système enzymatique soluble capable de transformer le NADH de la solution en un composé non susceptible de donner lieu à l'émission de photons en présence du système de bioluminescence est constitué de pyruvate et de lactate deshydrogénase (LDH), et

la première enzyme est la glucose-6-phosphate-deshydrogénase.

4. Procédé selon la revendication 3, caractérisé en ce que la lactate deshydrogénase est présente dans le milieu en large excès.

5. Procédé selon l'une des revendications 1 à 4, caractérisé en ce que pour le dosage d'un antigène ou d'un haptène :

a) on fait incuber ensemble :

l'échantillon à doser,

une solution de l'entité à doser marquée par la première enzyme, et

une solution d'anticorps spécifique de l'entité à doser,

b) on laisse réagir,

c) on ajoute une suspension de Sepharose portant un système permettant l'immobilisation de l'anticorps en solution et la ou les enzyme(s) du système de bioluminescence et un « anticorps universel », de préférence un anticorps anti-γ-globulines ou une protéine A,

d) on laisse de nouveau réagir,

e) on ajoute, en solution dans un tampon, les substrats d'enzymes autres que le composé nucléotide intermédiaire et les agents nécessaires à l'élimination du composé nucléotide intermédiaire en solution,

f) on laisse reposer jusqu'à obtention d'un signal stable de bioluminescence,

g) on effectue la lecture de bioluminescence et

h) on détermine la concentration de l'entité à doser au moyen d'un système d'étalonnage.

6. Procédé selon l'une des revendications 1 à 4, caractérisé en ce que pour le dosage d'un antigène :

a) on fait incuber ensemble :

l'échantillon à doser,

un anticorps spécifique de l'entité à doser, marqué par la première enzyme,

un support portant, immobilisés, un anticorps spécifique de l'entité à doser, de préférence différent du précédent, et la ou les enzyme(s) du système de bioluminescence,

b) on laisse incuber,

c) on ajoute, en solution dans un tampon, les substrats d'enzymes autres que le composé intermédiaire et les agents nécessaires à l'élimination du composé nucléotide intermédiaire en solution,

d) on laisse reposer jusqu'à obtention d'un signal stable de bioluminescence,

e) on effectue la lecture de bioluminescence et

f) on détermine la concentration de l'entité à doser au moyen d'un système d'étalonnage.

7. Réactif pour la mise en œuvre du procédé selon l'une des revendications 1 à 6, caractérisé en ce qu'il se présente sous la forme d'un solide, de préférence à l'état de microbilles de Sepharose, comportant, fixées à sa surface, une entité capable de former un complexe immunologique avec une autre entité et la ou les enzyme(s) d'un système de bioluminescence.

8. Réactif selon la revendication 7, caractérisé en ce que l'entité capable de former un complexe immunologique avec une autre entité est un « anticorps universel », de préférence un anticorps anti-γ-globulines ou une protéine A.

9. Réactif selon la revendication 7 ou 8, caractérisé en ce qu'il est constitué de microbilles de Sepharose portant, fixés à leur surface sur les mêmes microbilles ou sur des microbilles différentes, un « anticorps universel », de préférence un anticorps anti-γ-globulines ou une protéine A, une luciférase d'origine bactérienne et une oxydoréductase.

10. Réactif selon la revendication 7, caractérisé en ce qu'il est constitué de microbilles de Sepharose portant, fixés à leur surface, sur les mêmes microbilles ou sur des microbilles différentes, un anticorps spécifique de l'antigène à doser, une luciférase d'origine bactérienne et une oxydoréductase.

11. Nécessaire pour la mise en œuvre du procédé selon l'une des revendications 1 à 6, comprenant :

le réactif selon l'une des revendications 7 à 10, et

un flacon contenant l'entité à doser ou une entité spécifique de celle-ci, marquée par une enzyme.

12. Nécessaire pour la mise en œuvre du procédé selon l'une des revendications 1 à 4 ou 6, caractérisé en ce qu'il comprend :

un flacon contenant, à l'abri de la lumière, du Sepharose, en suspension dans l'eau ou sous forme de comprimé délitable dans l'eau, muni à sa surface d'un « anticorps universel », d'une luciférase d'origine bactérienne et d'une oxydoréductase,

un flacon contenant un anticorps spécifique de l'antigène à doser,

un flacon contenant l'antigène à doser marqué par une oxydoréductase, et

du tampon, du NAD ou du NADP, du FMN, un aldéhyde à longue chaîne, un système capable de détruire le NADH ou le NADPH en solution ainsi que le substrat de l'oxydoréductase servant de marqueur.

13. Nécessaire pour la mise en œuvre du procédé selon l'une des revendications 1 à 5, caractérisé en ce qu'il comprend :

un flacon contenant, à l'abri de la lumière, du Sepharose en suspension dans l'eau ou sous forme de comprimé délitable dans l'eau, muni à sa surface d'une entité spécifique de l'entité à doser, d'une luciférase d'origine bactérienne et d'une oxydoréductase,

un flacon contenant l'entité à doser ou une entité spécifique de l'entité à doser, marquée par une oxydoréductase et

du tampon, du NAD ou du NADP, du FMN, un aldéhyde à longue chaîne, un système capable de détruire le NADH ou le NADPH en solution, ainsi que le substrat de l'oxydoréductase servant de marqueur.

## Claims

1. Process for the immunoenzymatic dosage, in an aqueous medium, notably biological, of an entity X capable of forming an immunological complex with an entity Y, specific to the entity X, according to which the concentration of at least two enzymes is carried out at the level of a support by means of an immunological reaction in which the entities X and Y participate, thanks to which an intermediate compound is formed at the level of the support by a first enzyme and according to which this intermediate compound serves as a substrate for a second enzyme in order to give another compound which, directly or indirectly, produces, at support level, a measurable phenomenon, in conditions such that a relation may be established between the result of the measurement of this phenomenon and the concentration of entity X, characterized in that the support is porous and in that the said process essentially includes the stages consisting of :

a) bringing a first enzyme, as a marker of the entity X or the entity Y, by means of an immunological reaction of « competition » or « sandwich » type, between the entities X and Y, at the level of the porous support on which is fixed, in a preliminary or concomitant way, the entity Y, in order to produce on it, starting with a nucleotide compound introduced into the medium as a reagent, another, intermediate, nucleotide compound, capable of undergoing, on contact with the constituents of a bioluminescence system containing one or more enzymes of which one at least is also fixed on the support, a conversion leading to the emission of light, in conditions such that a relation may be established between the light emitted and the concentration of entity X in the aqueous medium, this medium having in addition added to it a system, preferably a soluble enzymatic system, capable of converting the intermediate nucleotide compound present in the solution into a compound not capable of emitting photons in the presence of the bioluminescence system, without modifying in a significant way the intermediate nucleotide compound formed at the support level,

b) leaving to rest until a stable signal is obtained,

c) measuring the light emitted and

d) starting with the measurement carried out, determining the concentration of entity X in the aqueous medium or the sample studied, using a calibrated system.

2. Process according to claim 1, characterized in that the intermediate nucleotide compound is chosen from ATP, NADH or NADPH.

3. Process according to claim 2, characterized in that :

the luciferase used is of bacterial origin,

the intermediate nucleotide compound is NADH,

the soluble enzymatic system capable of converting the NADH of the solution into a compound not capable of emitting photons in the presence of the bioluminescence system is constituted by pyruvate and lactate dehydrogenase (LDH), and

the first enzyme is glucose-6-phosphate-dehydrogenase.

4. Process according to claim 3, characterized in that the lactate dehydrogenase is present in the medium to a large excess.

5. Process according to one of claims 1 to 4, characterized in that for the dosage of an antigen or a haptene :

a) the following are incubated together :

the sample to be dosed,

a solution of the entity to be dosed marked with the first enzyme, and

a solution of antibody specific to the entity to be dosed,

b) the are left to react,

c) a suspension of Sepharose is added carrying a system which permits the immobilization of the antibody in solution and the enzyme or enzymes of the bioluminescence system and a « universal antibody », preferably an anti-gamma-globulins antibody or a protein A,

d) they are left to react again,

e) the enzyme substrates are added, in solution with a buffer, other than the intermediate nucleotide compound and the agents necessary for the elimination of the intermediate nucleotide compound in solution,

f) they are left to rest until a stable signal of bioluminescence is obtained,

g) the reading of the bioluminescence is carried out and

h) the concentration of the entity to be dosed is determined by means of a calibrated system.

6. Process according to one of claim 1 to 4, characterized in that for the dosage of an antigen :

a) the following are incubated together :

the sample to be dosed,

an antibody specific to the entity to be dosed, marked with the first enzyme,

a support carrying, immobilized, an antibody specific to the entity to be dosed, preferably different to the previous one, and the enzyme or enzymes of the bioluminescence system,

b) they are left to incubate,

c) the enzyme substrates are added, in solution with a buffer, other than the intermediate compound and the agents necessary for the elimination of the intermediate nucleotide compound in solution,

d) they are left to rest until a stable signal of bioluminescence is obtained,

e) the reading of bioluminescence is carried out and

f) the concentration of the entity to be dosed is determined by means of a calibrated system.

7. Reagent for putting to work the process according to one of claims 1 to 6, characterized in that it is presented in the form of a solid, preferably in the state of microballs of Sepharose, containing, fixed to its surface, an entity capable of forming an immunological complex with another entity and the enzyme or enzymes of a bioluminescence system.

8. Reagent according to claim 7, characterized in that the entity capable of forming an immunological complex with another entity is a « universal antibody », preferably an anti-gamma-globulins antibody or a protein A.

9. Reagent according to claim 7 or 8, characterized in that it is constituted by microballs of Sepharose carrying, fixed to their surface on the same microballs or on different microballs, a « universal antibody », preferably an anti-gamma-globulins antibody or a protein A, a luciferase of bacterial origin and an oxido-reductase.

10. Reagent according claim 7, characterized in that it is constituted by microballs of Sepharose carrying, fixed to their surface, on the same microballs or on different microballs, an antibody specific to the antigen to be dosed, a luciferase of bacterial origin and an oxido-reductase.

11. Kit for putting to work the process according to one of claims 1 to 6, including :

the reagent according to one of claims 7 to 10, and

a flask containing the entity to be dosed or a specific entity of this latter, marked with an enzyme.

12. Kit for putting to work the process according to one of claims 1 to 4 or 6, characterized in that it includes :

a flask, shaded from the light, containing Sepharose, in suspension in water or in the form of a tablet soluble in water, provided on its surface with a « universal antibody », with a luciferase of bacterial origin and with an oxido-reductase,

a flask containing an antibody specific to the antigen to be dosed,

a flask containing the antigen to be dosed marked with an oxido-reductase, and

a buffer, NAD or NADP, FMN, a long-chain aldehyde, a system capable of destroying the NADH or NADPH in solution as well as the oxido-reductase substrate serving as a marker.

13. Kit for putting to work the process according to one of claims 1 to 5, characterized in that it includes :

a flask, shaded from the light, containing Sepharose in suspension in water or in the form of a tablet disintegratable in water, provided on its surface with an entity specific to the entity to be dosed, with a luciferase of bacterial origin and with an oxido-reductase,

a flask containing the entity to be dosed or an entity specific to the entity to be dosed, marked with an oxido-reductase and

a buffer, NAD or NADP, FMN, a long-chain aldehyde, a system capable of destroying the NADH or NADPH in solution, as well as the oxido-reductase substrate serving as a marker.

**Patentansprüche**

1. Verfahren zur immunoenzymatischen Bestimmung einer Substanz X, die mit einer Substanz Y, die für die Substanz X spezifisch ist, einen immunologischen Komplex bilden kann, in einem wäßrigen, insbesondere biologischen Milieu, nach dem man die Anreicherung von mindestens zwei Enzymen an einem Träger mittels einer immunologischen Reaktion bewirkt, an der die Substanzen X und Y teilnehmen, wodurch am Träger eine intermediäre Verbindung durch ein erstes Enzym gebildet wird, und nach dem diese intermediäre Verbindung als Substrat für ein zweites Enzym dient, um eine andere Verbindung zu ergeben, die, direkt oder indirekt, am Träger eine meßbare Erscheinung bewirkt, unter Bedingungen, bei denen eine Relation hergestellt werden kann zwischen dem Ergebnis der Messung dieser Erscheinung und der Konzentration der Substanz X, dadurch gekennzeichnet, daß der Träger porös ist, und das Verfahren im wesentlichen aus den Stufen besteht :

a) Einbringen eines ersten Enzyms als Marker der Substanz X oder der Substanz Y mittels einer immunologischen Reaktion vom Typ « Competition » oder « Sandwich » zwischen die Substanzen X und Y an einem porösen Träger, auf dem vorhergehend oder gleichzeitig die Substanz Y gebunden wird, um dort aus einer Nucleotidverbindung, die als Reagenz in das Milieu eingeführt wird, eine andere intermediäre Nucleotidverbindung zu bilden, die dazu fähig ist, in Kontakt mit den Bestandteilen eines Biolumineszenzsystems, das ein oder mehrere Enzyme trägt, und von denen mindestens eines ebenfalls

auf dem Träger gebunden ist, eine Umwandlung zu ergeben, die zu einer Lichtemission führt, unter Bedingungen, bei denen eine Relation hergestellt werden kann zwischen dem emittierten Licht und der Konzentration der Substanz X im wäßrigen Milieu, wobei dieses Milieu außerdem einem System zugegeben wird, vorzugsweise einem löslichen enzymatischen System, das dazu fähig ist, die intermediäre, in der Lösung vorhandene Nucleotidverbindung in eine Verbindung überzuführen, die nicht dazu fähig ist, in Gegenwart des Biolumineszenzsystems eine Photonenemission zu ergeben, ohne auf wesentliche Weise die am Träger gebildete Nucleotidverbindung zu modifizieren,

b) Ruhen lassen bis zum Erhalt eines stabilen Signals,

c) Messung des emittierten Lichtes, und

d) Bestimmung der Konzentration der Substanz X in dem wäßrigen Milieu oder der zu untersuchenden Probe mit Hilfe eines Eichsystems, ausgehend von der durchgeführten Messung.

2. Verfahren nach Anspruch 1, dadurch gekennzeichnet, daß die intermediäre Nucleotidverbindung ausgewählt ist unter ATP, NADH oder NADPH.

3. Verfahren nach Anspruch 2, dadurch gekennzeichnet, daß :

die verwendete Luciferase bakteriellen Ursprungs ist,

die intermediäre Nucleotidverbindung NADH ist,

das lösliche enzymatische System, das zur Umwandlung von NADH in der Lösung in eine Verbindung, die nicht dazu fähig ist, in Gegenwart des Biolumineszenzsystems eine Photonenemission zu ergeben, aus Pyruvat und Lactatdehydrogenase (LDH) besteht, und

das erste Enzym Glucose-6-phosphat-dehydrogenase ist.

4. Verfahren nach Anspruch 3, dadurch gekennzeichnet, daß die Lactatdehydrogenase in dem Milieu in großem Überschuß vorhanden ist.

5. Verfahren nach einem der Ansprüche 1 bis 4, dadurch gekennzeichnet, daß man zur Bestimmung eines Antigens oder eines Haptens :

a) die Mischung inkubiert aus :

der zu bestimmenden Probe,

einer Lösung der zu bestimmenden Substanz, die durch das erste Enzym markiert ist, und

eine Lösung des für die zu bestimmende Substanz spezifischen Antikörpers,

b) Reagieren läßt,

c) eine Suspension von Sepharose, die ein System trägt, das die Immobilisierung des Antikörpers in Lösung erlaubt, und das oder die Enzyme des Lumineszenzsystems und einen « universellen Antikörper », vorzugsweise einen Anti-γ-globulin-Antikörper oder ein Protein A, zugibt,

d) erneut Reagieren läßt,

e) in einer Pufferlösung die Substrate von Enzymen, die von der intermediären Nucleotidverbindung und den zur Eliminierung der intermediären Nucleotidverbindung in Lösung notwendigen Reagenzien verschieden sind, zugibt,

f) bis zum Erhalt eines stabilen Biolumineszenz-Signals ruhen läßt,

g) die Ablesung der Biolumineszenz durchführt, und

h) die Konzentration der zu bestimmenden Substanz mittels eines Eichsystems bestimmt.

6. Verfahren nach einem der Ansprüche 1 bis 4, dadurch gekennzeichnet, daß man zur Bestimmung eines Antigens :

a) eine Mischung inkubiert aus :

der zu bestimmenden Probe,

eines für die zu bestimmende Substanz spezifischen Antikörpers, der durch das erste Enzym markiert ist,

einem Träger, der in immobilisierter Form einen für die zu bestimmende Substanz spezifischen Antikörper, der vorzugsweise verschieden ist von dem vorhergehenden, und das oder die Enzyme des Biolumineszenzsystems trägt,

b) Inkubieren läßt,

c) in einer Pufferlösung die Substrate von Enzymen, die von der intermediären Verbindung und den zur Eliminierung der intermediären Nucleotidverbindung in Lösung notwendigen Reagenzien verschieden sind, zugibt,

d) Ruhen läßt, bis ein stabiles Biolumineszenz-Signal erhalten wird,

e) die Ablesung der Biolumineszenz vornimmt, und

f) die Konzentration der zu bestimmenden Substanz mittels eines Eichsystems bestimmt.

7. Reagenz zur Durchführung des Verfahrens gemäß einem der Ansprüche 1 bis 6, dadurch gekennzeichnet, daß es in Form eines Feststoffs vorliegt, vorzugsweise in Form von Mikrokügelchen von Sepharose, die, an ihrer Oberfläche eine Substanz gebunden enthalten, die zur Bildung eines immunologischen Komplexes mit einer anderen Substanz oder dem oder den Enzymen des Biolumineszenzsystems fähig ist.

8. Reagenz nach Anspruch 7, dadurch gekennzeichnet, daß die zur Bildung eines immunologischen Komplexes mit einer anderen Substanz fähige Substanz ein « universeller Antikörper » ist, vorzugsweise ein Anti-γ-Globulin-Antikörper oder ein Protein A.

9. Reagenz nach Anspruch 7 oder 8, dadurch gekennzeichnet, daß es aus Mikrokügelchen von Sepharose besteht, die an ihrer Oberfläche an gleichen oder verschiedenen Mikrokügelchen einen

« universellen Antikörper », vorzugsweise einen Anti-γ-Globulin-Antikörper oder ein Protein A, eine Luciferase bakteriellen Ursprungs und eine Oxydoreductase gebunden enthält.

10. Reagenz nach Anspruch 7, dadurch gekennzeichnet, daß es aus Mikrokügelchen von Sepharose besteht, die an ihrer Oberfläche an gleichen oder verschiedenen Mikrokügelchen einen für das zu bestimmende Antigen spezifischen Antikörper, eine Luciferase bakteriellen Ursprungs und eine Oxydoreductase gebunden trägt.

11. Necessaire (Kit) zur Durchführung des Verfahrens nach einem der Ansprüche 1 bis 6, umfassend :

— das Reagenz gemäß einem der Ansprüche 7 bis 10, und

— ein Fläschchen, das die zu bestimmende Substanz oder eine dafür spezifische Substanz enthält, markiert durch ein Enzym.

12. Necessaire (Kit) zur Durchführung des Verfahrens nach einem der Ansprüche 1 bis 4 oder 6, dadurch gekennzeichnet, daß es umfaßt :

— ein Fläschchen, das, unter Lichtausschluß, Sepharose in Suspension in Wasser oder in Form von in Wasser zerfallenden Tabletten enthält, der an seiner Oberfläche mit einem « universellen Antikörper », einer Luciferase bakteriellen Ursprungs und einer Oxydoreductase ausgestattet ist,

— ein Fläschchen, das einen für das zu bestimmende Antigen spezifischen Antikörper enthält,

— ein Fläschchen, das den zu bestimmenden Antikörper, markiert durch eine Oxydoreductase, enthält, und

— einen Puffer, NAD oder NADP, FMN, einen langkettigen Aldehyd, ein System, das zur Zerstörung des NADH oder des NADPH in Lösung fähig ist, sowie das Substrat der als Marker dienenden Oxydoreductase.

13. Necessaire (Kit) zur Durchführung des Verfahrens nach einem der Ansprüche 1 bis 5, dadurch gekennzeichnet, daß es umfaßt :

— ein Fläschchen, das, unter Lichtausschluß, Sepharose in wäßriger Suspension oder in Form von in Wasser zerfallender Tabletten enthält, der an seiner Oberfläche mit einer für die zu bestimmende Substanz spezifischen Substanz, einer Luciferase bakteriellen Ursprungs und einer Oxydoreductase ausgestattet ist,

— ein Fläschchen, das die zu bestimmende Substanz oder eine für die zu bestimmende Substanz spezifische Substanz enthält, durch eine Oxydoreductase markiert, und

— einen Puffer, NAD oder NADP, FMN, einen langkettigen Aldehyd, ein System, das zur Zerstörung des NADH oder des NADPH in Lösung fähig ist, sowie das Substrat der als Marker dienenden Oxydoreductase.